# EUROPEAN PATENT APPLICATION

(11) **EP 4 649 922 A1**
(43) Date of publication of application: **19.11.2025**
(21) Application number: 23915807.4
(22) Date of filing: 26.12.2023
(51) Int. Cl.: A61F 2/38

(54) **KNEE JOINT PAD AND KNEE JOINT PROSTHESIS HAVING SAME**

(30) Priority: 13.01.2023 CN 202310074377
(71) Applicant: Beijing Naton Medical Technology Holdings Co., Ltd., Beijing 100094 (CN); Tianjin Zhengtian Medical Instrument Co., Ltd., Airport Economic Zone Tianjin, 300308 (CN)
(72) Inventor: WANG, Jian, Beijing 100094 (CN); SONG, Dayong, Beijing 100094 (CN); XU, Shufu, Beijing 100094 (CN); DING, Yubao, Beijing 100094 (CN)
(74) Representative: advotec.
(86) International application number: PCT/CN2023/141772
(87) International publication number: WO 2024/149054

(57) **Abstract**

Provided are a knee joint pad and a knee joint prosthesis having same. The knee joint pad comprises a pad body. A medial condyle supporting surface and a lateral condyle supporting surface which are used for matching a corresponding femoral condyle are formed on the pad body; both the medial condyle supporting surface and the lateral condyle supporting surface are concave curved surfaces; the lateral condyle supporting surface comprises a first external curved surface portion, a linear surface portion and a second external curved surface portion which are sequentially arranged in the front-back direction of the lateral condyle supporting surface; each of the first external curved surface portion and the second external curved surface portion is smoothly connected to the linear surface portion; when the femoral condyle flexes on the pad body, the lateral condyle of the femoral condyle can slide on the linear surface portion; the first external curved surface portion is used for matching the lateral condyle of the femoral condyle and keeping a human body in a standing state.

## Description

### CROSS-REFERENCE TO RELATED APPLICATIONS

The application is based on and claims priority to Chinese Patent Application No. 2023100743770, filed on January 13, 2023, the entire content of which is incorporated herein by reference.

### FIELD

The present disclosure relates to the technical field of medical prosthesis, specifically to a knee-joint pad and a knee-joint prosthesis with same.

### BACKGROUND

Knee joint replacement is to use artificial knee joint prosthesis to replace the diseased articular cartilage and meniscus while retaining normal articular ligaments and other tissue knee joint prosthesis, where a knee joint pad is placed between femur and tibia to have a contact, with a high-level fit, within the femoral component and tibial component, and to coordinate the rotation of femur and tibia, such that the human body can move naturally within a certain range after surgery. In the flexion from 5° (a standing state) to 120° (a flexion state) of knee joint of normal person, the femur rotates outward relative to tibia with condyles medialis as axis during the flexion from extension position to flexion position (120°) of knee joint.

In the related art, a condyles lateralis bearing surface of a knee-joint pad is generally designed as an arc-shaped concave surface with a single radius. However, when the lateral condyle of the femur is fitted to the condyles lateralis bearing surface, with the human body moving from a standing state to a flexion state, such a single-radius design may cause the condyles lateralis prosthesis of the femur to be restricted by a higher rear side of the lateral pad of the tibia, so that the femoral prosthesis cannot roll back further, thereby negatively affecting the high flexion performance of the prosthesis.

### SUMMARY

Embodiments of the present disclosure are intended to solve one of the technical problems in the related art at least to some extent. To this end, embodiments of the present disclosure provide a knee-joint pad. The knee-joint pad has the advantages of high flexibility in flexion and extension activities and long service life.

The present disclosure further provides in embodiments a knee-joint prosthesis.

According to embodiments of the present disclosure, the knee-joint pad includes a pad body.

The pad body is formed with a condyles medialis bearing surface and a condyles lateralis bearing surface configured to be fitted to a corresponding femural condyle, where the condyles medialis bearing surface and the condyles lateralis bearing surface are each concavely curved, the condyles lateralis bearing surface, along a front-rear direction of the condyles lateralis bearing surface, is provided with a first lateralis curved surface portion, a straight portion and a second lateralis curved surface portion set from front to rear, each of the first lateralis curved surface portion and the second lateralis curved surface portion is smoothly connected with the straight portion, and a condyles lateralis of the femural condyle is slidable on the straight portion when the femural condyle is flexed on the pad body, and the first lateralis curved surface portion is configured to be fitted to the condyles lateralis of the femural condyle under a standing state of the human body.

According to embodiments of the present disclosure, the knee-joint pad divides the condyles lateralis bearing surface, along the front-rear direction of the condyles lateralis bearing surface, into the first lateralis curved surface portion, the straight portion and the second lateralis curved surface portion set from front to rear, and each of the first lateralis curved surface portion and the second lateralis curved surface portion is smoothly connected with the straight portion, fitting to a condylar surface of the femural condyle under a standing state of the human body through the straight portion, thus enabling the knee joint to have better slide. Due to physiological kinematics of "shifting with the medial condyle as axis, the lateral condyle rolling backward" in knee-joint flexion, the lateral condyle may roll and slide along an arc trajectory of the condyles lateralis bearing surface under different knee-joint flexion angles. Accordingly, when the femoral condyle flexes on the knee joint pad, the lateral condyle can slide backward on this lateral straight portion, so as to ensure the relative stability of the lateral condyle and the safety of the tibial prosthesis. It makes it easier to bend one's knees, and more in line with the movement of the human body's joints, thereby improving the flexibility of flexion and extension activities of the human body with knee replacement surgery. In addition, it can further reduce the abrasion between the knee joint pad and the lateral condyle surface, thus benefiting prolonging the service life of the knee joint pad.

The knee-joint pad according to embodiments of the present disclosure therefore has the advantages of high flexibility in flexion and extension activities and long service life.

In some embodiments, the straight portion is tangent to at least one of the first lateralis curved surface portion or the second lateralis curved surface portion.

In some embodiments, a length of the straight portion along the front and rear direction of the condyles lateralis bearing surface takes account for 1.5% to 38% of a length of the condyles lateralis bearing surface along the front-rear direction of the condyles lateralis bearing surface.

In some embodiments, the length of the straight portion along the front and rear direction of the condyles lateralis bearing surface is 1 mm to 12 mm.

In some embodiments, when the femural condyle is flexed by 60° to 90° on the pad body, the condyles lateralis of the femural condyle slides on the straight portion.

In some embodiments, when the femural condyle is flexed over 90° on the pad body, the condyles lateralis of the femural condyle fits to the second lateralis curved surface portion.

In some embodiments, the first lateralis curved surface portion includes: a first curved surface segment; and a second curved surface segment smoothly connected with the first curved surface segment, where the second curved surface segment is smoothly connected with the straight portion, the first curved surface segment has a greater curvature radius than the second curved surface segment, and the second curved surface segment is configured to be fitted to the condyles lateralis of the femural condyle under a standing state of the human body.

In some embodiments, the second lateralis curved surface portion has a greater curvature radius than the first curved surface segment.

In some embodiments, the condyles medialis bearing surface includes a first medialis curved surface portion, located at a middle of a curved surface groove, and configured to be fitted to the condyles medialis of the femural condyle under a standing state of the human body.

In some embodiments, the condyles medialis bearing surface further includes a second medialis curved surface portion configured to be fitted to the condyles medialis of the femural condyle, the second medialis curved surface portion is provided close to a front end of the human body relative to the first medialis curved surface portion, and the second medialis curved surface portion is connected with and smoothly transitions to the first medialis curved surface portion.

In some embodiments, the condyles medialis bearing surface further includes a third medialis curved surface portion configured to be fitted to the condyles medialis of the femural condyle, the third medialis curved surface portion is provided close to a rear end of the human body relative to the first medialis curved surface portion, and the third medialis curved surface portion is connected with and smoothly transitions to the first medialis curved surface portion.

In some embodiments, the second medialis curved surface portion has a greater curvature radius than the first medialis curved surface portion.

In some embodiments, the third medialis curved surface portion has a greater curvature radius than the first medialis curved surface portion.

In some embodiments, a horizontal height of a front end of the condyles medialis bearing surface is greater than that of a rear end of the condyles medialis bearing surface.

In some embodiments, a horizontal height of an edge of a front end of the condyles lateralis bearing surface is greater than a horizontal height of a rear end of the condyles lateralis bearing surface.

According to embodiments of the present disclosure, a knee-joint prosthesis may include: a knee-joint pad provided in any one embodiments above; a tibial plateau support, connected with the knee-joint pad and joinable with a tibia; and a femural condyle prosthesis, fitted with the knee-joint pad and connected with a femur.

### BRIEF DESCRIPTION OF THE DRAWINGS

Figure 1 is a front view of a knee-joint prosthesis according to an embodiment of the present disclosure.
Figure 2 is a sectional view along A-A in Figure 1.
Figure 3 is a sectional view along B-B in Figure 1.

Drawing reference:
pad body 100;
condyles medialis bearing surface 1; first medialis curved surface portion 11; second medialis curved surface portion 12; third medialis curved surface portion 13;
condyles lateralis bearing surface 2; first lateralis curved surface portion 21; first curved surface segment 211; second curved surface segment 212; straight portion 22; second lateralis curved surface portion 23.

### DETAILED DESCRIPTION

Reference will be made in detail to embodiments of the present disclosure. The embodiments described herein with reference to drawings are explanatory, illustrative, and used to generally understand the present disclosure. The embodiments shall not be construed to limit the present disclosure.

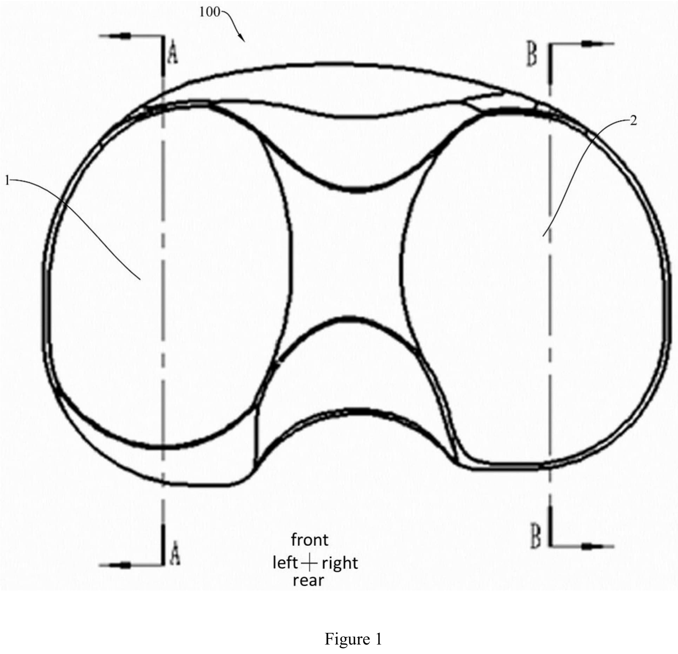

A knee-joint pad and knee-joint prosthesis with same according to embodiments of the present disclosure is described below with reference to Figure 1 to Figure 3.

In the field of anatomy and medical devices, the terms "medialis", "lateralis", "front (anterior)", "rear (posterior)", "distal", "proximal", "sagittal", "coronal", "cross-section", etc. for the orientation and planes have specific meanings and are well known to those skilled in the art. Unless otherwise specify, these terms refer to meanings generally recognized by those skilled in the art.

In general, when describing the human body, joint, or prosthesis, there are three views usually involved: sagittal plane, coronal plane, and cross-section. Sagittal plane refers to the longitudinal section that divides the human body or joint into left and right parts from a front to rear direction, where the sagittal plane passing through the middle of the human body is the median sagittal plane, which divides the human body into two equal parts on the left and right sides. Coronal plane refers to a longitudinal section that divides the body or joint into two parts from a left to right direction. The coronal plane is perpendicular to the sagittal plane. Cross section is a plane parallel to the ground plane that divides the human body or joint into upper and lower parts. The cross section is perpendicular to the coronal and sagittal planes.

It would be understood that when describing a knee joint or knee joint prosthesis, sagittal plane, coronal plane, or cross section refers to a section of a person standing normally upright, with the knee joint bent at an angle of 0°. With the knee joint or knee joint prosthesis extended or flexed, or the posture of the body adjusted, such sections may change accordingly.

In general, when describing the human body, joints, or prostheses, there are three different orientations involved: distal-proximal, medialis-lateralis, and front-rear. Distal end refers to the end of the human body or joint relatively far away from the trunk. Proximal end refers to the end of the human body or joint that is relatively close to the trunk. The term "medialis" refers to the side of the human body relatively close to the median sagittal plane, while "lateralis" refers to the side of the human body opposite the median sagittal plane. Front end refers to the end of the sagittal plane relatively close to the abdomen. Rear end refers to the end of the sagittal plane relatively close to the back.

In some specific embodiments, the present disclosure provides a knee-joint pad for replacing the lateral meniscus of a tibia in knee replacement surgery and fitting with the condyles medialis and condyles lateralis of the femur. It would be understood that the knee joint pad includes a left-leg knee-joint pad and a right-leg knee-joint pad respectively applied to the left leg and the right leg, where the left-leg knee-joint pad and the right-leg knee-joint pad are radially symmetrical with respect to the median sagittal plane of the human body.

According to embodiments of the present disclosure, the knee-joint pad includes a pad body 100, which is formed with a condyles medialis bearing surface 1 and a condyles lateralis bearing surface 2 configured to be fitted to a corresponding femural condyle, where the condyles medialis bearing surface 1 and the condyles lateralis bearing surface 2 are each concavely curved. The condyles lateralis bearing surface 2, along the front-rear direction of the condyles lateralis bearing surface as shown in Figure 1, is provided with a first lateralis curved surface portion 21, a straight portion 22 and a second lateralis curved surface portion 23 set from front to rear, where each of the first lateralis curved surface portion 21 and the second lateralis curved surface portion 23 is smoothly connected with the straight portion 22, and a condyles lateralis of the femural condyle is slidable on the straight portion 22 when the femural condyle is flexed on the pad body 100. The first lateralis curved surface portion 21 is configured to be fitted to the condyles lateralis of the femural condyle under a standing state of the human body. It should be noted that, each of the first lateralis curved surface portion 21 and the second lateralis curved surface portion 23 being smoothly connected with the straight portion 22 specifically means there is no stuck feeling when the knee-joint pad fits to the corresponding supporting femur. The straight portion 22 is configured to be fitted to the condyles lateralis of the femural condyle under a standing state of the human body, while the second lateralis curved surface portion 23 is configured to be fitted to the condyles lateralis of the femural condyle under a flexion state of the human body.

According to the research, when fitting to the medial condyle surface supporting the femur, a condyles medialis bearing surface 1 will rotate around the medial side as the center, and when fitting to the lateral condyle surface of the femur, a condyles lateralis bearing surface 2 will roll and relatively slide, so that the human body changing from the standing state to the flexion state will cause the condyles lateralis of the femur to be restricted by a higher rear side of the keen joint pad of the tibia, leading to the person with knee joint replacement having difficulty in moving from the standing state to the flexion state after surgery. That affects the flexibility on flexion of the human body.

According to embodiments of the present disclosure, the knee-joint pad divides the condyles lateralis bearing surface 2, along a front-rear direction of the condyles lateralis bearing surface 2, into the first lateralis curved surface portion 21, the straight portion 22 and the second lateralis curved surface portion 23 set from front to rear, and each of the first lateralis curved surface portion 21 and the second lateralis curved surface portion 23 is smoothly connected with the straight portion 22, fitting to a condylar surface of the femural condyle under the standing state of the human body through the straight portion 22. Because the lateral condyle of knee joint moves a long distance when flexing 30° to 90°, the design of straight portion 22 is conducive to the sliding of femoral condyle, thereby improving the flexibility of flexion and extension. Further, the lateral condyle of the femur can roll and slide along an arc trajectory of the condyles lateralis bearing surface 2 under different knee-joint flexion angles, reappearing the physiological kinematics of "shifting with the medial condyle as axis, the lateral condyle rolling backward". Moreover, it makes it easier to bend one's knees, and more in line with the movement of the human body's joints. Accordingly, when the femoral condyle flexes on the knee joint pad, the lateral condyle can slide backward on this lateral straight portion, so as to ensure the relative stability for the rolling and sliding of the lateral condyle, and thus the safety of the human body during flexion and extension, thereby improving the flexibility of flexion and extension activities of the human body with knee replacement surgery. In addition, it can further reduce the abrasion between the knee joint pad and the lateral condyle surface, thus benefiting prolonging the service life of the knee joint pad.

The knee-joint pad according to embodiments of the present disclosure therefore has the advantages of high flexibility in flexion and extension activities and long service life.

In actual use, the condyles medialis bearing surface 1 and the condyles lateralis bearing surface 2 are provided independently from each other along the left-right direction of the human body (e.g., in the left-right direction shown in Figure 1), the condyles medialis bearing surface 1 is positioned medial to the condyles lateralis bearing surface 2, and by providing the two independent bearing surfaces (i.e., the condyles medialis bearing surface 1 and the condyles lateralis bearing surface 2) for the medial and lateral condyles respectively, where the condyles medialis bearing surface 1 is fitted to the medial condyle of the femoral condyle, the medial condyle of the femoral condyle rotates about an axis relative to the condyles medialis bearing surface 1, and the lateral condyle may roll and slide along an arc trajectory of the condyles lateralis bearing surface 2 under different knee-joint flexion angles.

It will be appreciated that the knee joint pad shown in Figure 1 actually is suitable and corresponds to the right knee joint of a person, while the knee joint pad corresponding to the left knee joint is a mirror image of the knee joint pad as shown in the Figure. Furthermore, the knee joint pad is available in a variety of size specifications to suit the needs of different patients.

Sectioning position of cross section B-B corresponds to the extent of the contact path during the fit of the lateral articular surface to the lateral condyle. The movement path of lateral condyle refers to a collection of contact points between lateral condyle and the lateral articular surface during flexion, that is, the trajectory of lateral condyle moving on lateral articular surface during flexion of the human body.

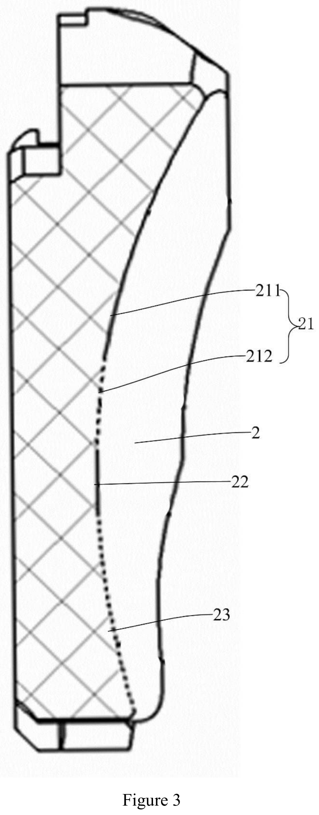

As shown in Figure 1 and Figure 3, the straight portion 22 is tangent to at least one of the first lateralis curved surface portion 21 or the second lateralis curved surface portion 23. In other words, the straight portion 22 is tangent to the first lateralis curved surface portion 21; or, the straight portion 22 is tangent to the second lateralis curved surface portion; or the straight portion 22 is tangent to each of the first lateralis curved surface portion 21 and the second lateralis curved surface portion 23.

According to embodiments of the present disclosure, in the knee-joint pad, the straight portion 22 is tangent to at least one of the first lateralis curved surface portion 21 or the second lateralis curved surface portion 23, such that the condyles lateralis bearing surface 2 can fit to and support the sliding movement of the lateral condyle surface supporting the femur better, thereby further improving the flexibility of flexion and extension activities of the human body with knee replacement surgery.

A length of the straight portion 22 along the front and rear direction of the condyles lateralis bearing surface 2 takes account for 1.5% to 38% of a length of the condyles lateralis bearing surface 2 along the front-rear direction of the condyles lateralis bearing surface 2.

According to embodiments of the present disclosure, in the knee-joint pad, the length of the straight portion 22 along the front and rear direction of the condyles lateralis bearing surface 2 takes account for 1.5% to 38% of the length of the condyles lateralis bearing surface 2 along the front-rear direction of the condyles lateralis bearing surface 2, thereby avoiding not only the length of the straight portion 22 being too short to limit the slide distance of the lateral condyle and thus limit knee-joint flexion angles, but also the length of the straight portion 22 being too long to reduce the deep flexion restriction of the lateral condyle and thus destabilize the joint or increase the wear of the rear edge.

In some embodiments, the length of the straight portion 22 along the front and rear direction of the condyles lateralis bearing surface 2 is 1 mm to 12 mm, thereby avoiding not only the length of the straight portion 22 being too short to limit the slide distance of the lateral condyle and thus limit knee-joint flexion angles, but also the length of the straight portion 22 being too long to reduce the deep flexion restriction of the lateral condyle and thus destabilize the joint or increase the wear of the rear edge.

For example, the length of the straight portion 22 along the front and rear direction of the condyles lateralis bearing surface 2 may be 1.0 mm, 1.5 mm, 2.0 mm, 2.5 mm, 3.0 mm, 3.5 mm, 4.0 mm, 4.5 mm, 5.0 mm, 6 mm, 6.5 mm, 7.0 mm, 7.5 mm, 8.0 mm, 8.5 mm, 9.0 mm, 9.5 mm, 10.0 mm, 10.5 mm, 11.0 mm, 11.5 mm or 12 mm.

When the femural condyle is flexed by 60° to 90° on the pad body 100, the condyles lateralis of the femural condyle slides on the straight portion 22, which is closer to the way the knee moves naturally.

When the femural condyle is flexed over 90° on the pad body 100, the condyles lateralis of the femural condyle fits to the second lateralis curved surface portion 23.

As shown in Figure 1 and Figure 3, the first lateralis curved surface portion 21 includes: a first curved surface segment 211; and a second curved surface segment 212 smoothly connected with the first curved surface segment 211, where the second curved surface segment 212 is smoothly connected with the straight portion 22, and the first curved surface segment 211 has a greater curvature radius than the second curved surface segment 212. It would be understood that the condyles lateralis bearing surface 2, along the front-rear direction of the condyles lateralis bearing surface 2, is provided with the first curved surface segment 211, the second curved surface segment 212, the straight portion 22 and the second lateralis curved surface portion 23 set from front to rear, where the first medialis curved surface portion 211 is provided close to a front end of the human body relative to the second medialis curved surface portion 212, and is coordinated, during a deep flexion, with the second lateralis curved surface portion 23 on the lateral condyle surface supporting the femur.

According to embodiments of the present disclosure, in the knee-joint pad, the first lateralis curved surface portion 21 is divided into the first curved surface segment 211 and the second curved surface segment 212, and the first curved surface segment 211 has a greater curvature radius than the second curved surface segment 212, which effectively reduces the restriction on deep flexion of lateral condyle, thereby achieving deep bending more easily.

It should be noted that, the second lateralis curved surface portion may also include multiple curved surface segments.

As shown in Figure 1 and Figure 3, the second lateralis curved surface portion 23 has a greater curvature radius than the first curved surface segment 211. It would be understood that the second lateralis curved surface portion 23 has a greater curvature radius than the first curved surface segment 211, and the first curved surface segment 211 has a greater curvature radius than the second curved surface segment 212.

According to embodiments of the present disclosure, in the knee-joint pad, the second lateralis curved surface portion 23 has a greater curvature radius than the first curved surface segment 211, and the first curved surface segment 211 has a greater curvature radius than the second curved surface segment 212, which reduces the restriction on femoral condyle as well as the wear.

Accordingly, in the flexion and extension of the human body, the lateral condyle surface of the femur can rotate with respect to the condyles lateralis bearing surface 2, providing patients with more natural knee joint function.

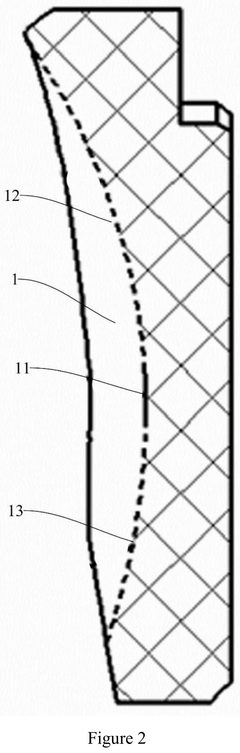

As shown in Figure 1 and Figure 2, the condyles medialis bearing surface 1 includes a first medialis curved surface portion 11, located at a middle of a curved surface groove and configured to be fitted to the condyles surface of the femural condyle under the standing state of the human body.

The condyles medialis bearing surface 1 is represented by the cross section A-A in Figure 2, sectioning position of which corresponds to the extent of the contact path during the fit of the medial articular surface to the medial condyle. The movement path of medial condyle refers to a collection of contact points between medial condyle and the medial articular surface during flexion, that is, the trajectory of medial condyle moving on medial articular surface during flexion of the human body. In some embodiments, the point of the first medialis curved surface portion 11 is also the lowest point of the medial articular surface on the coronal plane, thereby improving the rotational stability of the knee joint pad and the corresponding condyle surface of the fitted femur.

In some embodiments, as shown in Figure 1 and Figure 2, the condyles medialis bearing surface 1 is generally a ball-and-socket joint surface, thereby improving the rotational stability of the knee joint pad and the corresponding condyle surface of the fitted femur.

In some embodiments, the condyles medialis bearing surface 1 may have a concave depth for 6 mm-12 mm.

As shown in Figure 1 and Figure 2, the condyles medialis bearing surface 1 further includes a second medialis curved surface portion 12 configured to be fitted to the condyles medialis of the femural condyle, where the second medialis curved surface portion 12 is provided close to a front end of the human body relative to the first medialis curved surface portion 11, and the second medialis curved surface portion 12 is connected with and smoothly transitions to the first medialis curved surface portion 11.

As shown in Figure 1 and Figure 2, the second medialis curved surface portion 12 has a greater curvature radius than the first medialis curved surface portion 11, which reduces the restriction on femoral condyle during the flexion of knee joint, and is conductive to decrease pad wear.

As shown in Figure 1 and Figure 2, the third medialis curved surface portion 13 has a greater curvature radius than the first medialis curved surface portion 11. According to embodiments of the present disclosure, a knee joint prosthesis with the knee joint pad enables the medial condyle to roll back to a higher degree of flexion under deep flexion.

As shown in Figure 1 and Figure 2, a horizontal height of a front end of the condyles medialis bearing surface 1 is greater than that of a rear end of the condyles medialis bearing surface 1. This not only can effectively reduce the height of the most rear side of the condyles medialis bearing surface, so that the medial condyle can roll back to a higher degree of flexion under deep flexion of the knee joint pad thereby preventing the impact with the femoral shaft (thus presenting the advantages of improved high flexion performance of the condyles medialis bearing surface 1 and improved use effect), but also effectively ensure the rotational stability of the condyles medialis bearing surface 1 to avoid femoral dislocation.

As shown in Figure 1 and Figure 2, a horizontal height of an edge of a front end of the condyles lateralis bearing surface 2 is greater than a horizontal height of a rear end of the condyles lateralis bearing surface 2. This not only can effectively reduce the height of the most rear side of the condyles lateralis bearing surface 2, so that the medial condyle can roll back to a higher degree of flexion under deep flexion with the knee joint prosthesis thereby improving high flexion performance of the knee joint pad and use effect, but also effectively ensure the rotational stability of the knee joint pad to avoid femoral dislocation.

In some embodiments, the pad body may be a polyethylene pad, which has the advantages of strong bearing capacity and good wear performance.

According to embodiments of the present disclosure, a knee-joint prosthesis may include: a knee-joint pad provided in any one embodiments above; a tibial plateau support, connected with the knee-joint pad and joinable with a tibia; and a femural condyle prosthesis, fitted with the knee-joint pad and connected with a femur. The knee-joint prosthesis has the advantages of high flexibility in flexion and extension activities and long service life.

In the specification, it should be understood that, the terms indicating orientation or position relationship such as "central", "longitudinal", "lateral", "width", "thickness", "above", "below", "front", "rear", "right", "left", "vertical", "horizontal", "top", "bottom", "inner", "outer", "clockwise", "counter-clockwise", "axial", "radial", "circumferential" should be construed to refer to the orientation or position relationship as then described or as shown in the drawings. These terms are merely for convenience and concision of description and do not alone indicate or imply that the device or element referred to must have a particular orientation or must be configured or operated in a particular orientation. Thus, it cannot be understood to limit the present disclosure.

In addition, terms such as "first" and "second" are used herein for purposes of description and are not intended to indicate or imply relative importance or significance or impliedly indicate quantity of the technical feature referred to. Thus, the feature defined with "first" and "second" may comprise one or more this features. In the description of the present disclosure, "a plurality of" means two or more than two this features, unless specified otherwise.

In the present disclosure, unless specified or limited otherwise, the terms "mounted", "connected", "coupled", "fixed" and the like are used broadly, and may be, for example, fixed connections, detachable connections, or integrated connections; may also be mechanical or electrical connections or communicated with each other; may also be direct connections or indirect connections via intervening structures; may also be inner communications of two elements or mutual interaction between two elements, which can be understood by those skilled in the art according to specific situations, unless specified otherwise.

In the present disclosure, unless specified or limited otherwise, a structure in which a first feature is "on" or "below" a second feature may be an embodiment in which the first feature is in direct contact with the second feature, or an embodiment in which the first feature and the second feature are contacted indirectly via an intermediation. Furthermore, a first feature "on", "above" or "on top of" a second feature may include an embodiment in which the first feature is right or obliquely "on", "above" or "on top of" the second feature, or just means that the first feature is at a height higher than that of the second feature; while a first feature "below", "under" or "on bottom of" a second feature may include an embodiment in which the first feature is right or obliquely "below", "under" or "on bottom of" the second feature, or just means that the first feature is at a height lower than that of the second feature.

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example" or "in some examples", in various places throughout this specification are not necessarily referring to the same embodiment or example of the present disclosure. Furthermore, the particular features, structures, materials, or characteristics may be combined in any suitable manner in one or more embodiments or examples. Besides, any different embodiments and examples and any different characteristics of embodiments and examples may be combined by those skilled in the art without contradiction.

Although explanatory embodiments have been shown and described, it would be appreciated by those skilled in the art that the above embodiments cannot be construed to limit the present disclosure, and changes, alternatives, and modifications can be made in the embodiments in the scope of the present disclosure.

## Claims

1. A knee-joint pad, comprising:
a pad body, formed with a condyles medialis bearing surface and a condyles lateralis bearing surface configured to be fitted to a corresponding femural condyle, wherein
the condyles medialis bearing surface and the condyles lateralis bearing surface are each concavely curved,
the condyles lateralis bearing surface, along a front-rear direction of the condyles lateralis bearing surface, is provided with a first lateralis curved surface portion, a straight portion and a second lateralis curved surface portion set from front to rear,
each of the first lateralis curved surface portion and the second lateralis curved surface portion is smoothly connected with the straight portion, and a condyles lateralis of the femural condyle is slidable on the straight portion when the femural condyle is flexed on the pad body, and
the first lateralis curved surface portion is configured to be fitted to the condyles lateralis of the femural condyle under a standing state of the human body.

2. The knee-joint pad according to claim 1, wherein
the straight portion is tangent to at least one of the first lateralis curved surface portion or the second lateralis curved surface portion; and/or
a length of the straight portion along the front and rear direction of the condyles lateralis bearing surface takes account for 1.5% to 38% of a length of the condyles lateralis bearing surface along the front-rear direction of the condyles lateralis bearing surface; and/or
the length of the straight portion along the front-rear direction of the condyles lateralis bearing surface is 1 mm to 12 mm.

3. The knee-joint pad according to claim 1 or 2, wherein
when the femural condyle is flexed by 60° to 90° on the pad body, the condyles lateralis of the femural condyle slides on the straight portion, and/or
when the femural condyle is flexed over 90° on the pad body, the condyles lateralis of the femural condyle fits to the second lateralis curved surface portion.

4. The knee-joint pad according to any one of claims 1 to 3, wherein the first lateralis curved surface portion comprises:
a first curved surface segment; and
a second curved surface segment smoothly connected with the first curved surface segment,
wherein the second curved surface segment is smoothly connected with the straight portion, the first curved surface segment has a greater curvature radius than the second curved surface segment, and the second curved surface segment is configured to be fitted to the condyles lateralis of the femural condyle under a standing state of the human body.

5. The knee-joint pad according to claim 4, wherein the second lateralis curved surface portion has a greater curvature radius than the first curved surface segment.

6. The knee-joint pad according to any one of claims 1 to 5, wherein the condyles medialis bearing surface comprises a first medialis curved surface portion, located at a middle of a curved surface groove, and configured to be fitted to the condyles medialis of the femural condyle under a standing state of the human body.

7. The knee-joint pad according to claim 6, wherein
the condyles medialis bearing surface further comprises a second medialis curved surface portion configured to be fitted to the condyles medialis of the femural condyle, the second medialis curved surface portion is provided close to a front end of the human body relative to the first medialis curved surface portion, and the second medialis curved surface portion is connected with and smoothly transitions to the first medialis curved surface portion, and/or
the condyles medialis bearing surface further comprises a third medialis curved surface portion configured to be fitted to the condyles medialis of the femural condyle, the third medialis curved surface portion is provided close to a rear end of the human body relative to the first medialis curved surface portion, and the third medialis curved surface portion is connected with and smoothly transitions to the first medialis curved surface portion.

8. The knee-joint pad according to claim 7, wherein
the second medialis curved surface portion has a greater curvature radius than the first medialis curved surface portion, and/or
the third medialis curved surface portion has a greater curvature radius than the first medialis curved surface portion.

9. The knee-joint pad according to any one of claims 1 to 8, wherein
a horizontal height of a front end of the condyles medialis bearing surface is greater than that of a rear end of the condyles medialis bearing surface; and/or
a horizontal height of an edge of a front end of the condyles lateralis bearing surface is greater than a horizontal height of a rear end of the condyles lateralis bearing surface.

10. A knee-joint prosthesis, comprising:
a knee-joint pad of any one of claims 1 to 9;
a tibial plateau support, connected with the knee-joint pad and joinable with a tibia; and
a femural condyle prosthesis, fitted with the knee-joint pad and connected with a femur.
